# EUROPÄISCHE PATENTANMELDUNG

(11) **EP 4 850 745 A1**
(43) Veröffentlichungstag der Anmeldung: **09.12.2026**
(21) Anmeldenummer: 25180661.8
(22) Anmeldetag: 04.06.2025
(51) Int. Cl.: A61M 60/562, A61M 1/36, A61M 60/109, A61M 60/232, A61M 60/38, A61M 60/515, A61M 60/523, A61M 60/531, A61M 60/546

(54) **VORRICHTUNG ZUR ERZEUGUNG EINER EXTRAKORPORALEN ZIRKULATION MIT PULSATIVEM BLUTFLUSS**

(71) Anmelder: Justus-Liebig-Universität Gießen, 35390 Gießen (DE)
(72) Erfinder: KALDER, Johannes, 35580 Wetzlar (DE)
(74) Vertreter: Stumpf, Peter

(57) **Zusammenfassung**

Die Erfindung betrifft eine Vorrichtung (1) zur Erzeugung einer extrakorporalen Zirkulation mit pulsativem Blutfluss wenigstens umfassend
• ein Rohr (10), wobei das Rohr (10) dabei an einem Ende wenigstens eine erste Öffnung (110) und an dem dieses Ende gegensetzten Ende eine zweite Öffnung (120) aufweist, sodass sie an eine Herzlungenmaschinen anschließbar ist und Blut aufnehmen kann, sodass ein externen Blutdurchfluss durch das Rohr (10) und so eine extrakorporale Zirkulation erfolgen kann,
• ein Ventil (20), welcher innerhalb des Rohrs (10) angeordnet ist
• eine Pumpe (30), welches innerhalb des Rohrs (10) so angeordnet ist, dass sie die Flussrate und damit den Blutdruck des Blutes welches das Rohr (10) passiert verändern und so eine künstliche Systole erzeugen kann.
• ein Steuerelement (40), welches so ausgebildet ist, dass es die Pumpe (20) und das Ventil (30) im Rohr (10) steuern kann
Dabei umfasst das Rohr (10) ein Reservoir (130), welches zumindest abschnittsweise elastisch ausgebildet ist, sodass es Blut kurzzeitig aufstauen und zur Verfügung stellen kann, wenn Blut mit einer fluktuierenden Rate durch das Rohr (10) transportiert wird, wobei das Ventil (20) auf der der zweiten Öffnung (12) zugewandten Seite des Reservoirs (130) innerhalb der Rohr (10) angeordnet und so ausgebildet ist, dass durch den Grad der Öffnung des Ventils (20) die Menge des Blutes, welches durch die Rohr (10) fließt, gesteuert werden kann.

## Beschreibung

Die vorliegende Erfindung betrifft eine Vorrichtung zur Erzeugung einer extrakorporalen Zirkulation mit pulsativem Blutfluss.

### Beschreibung und Einleitung des allgemeinen Gebietes der Erfindung

Das menschliche Herz-Kreislauf-System ist ein geschlossener, unter Druck stehender hydraulischer Kreislauf, der aus Gefäßen besteht, die innen von Endothelzellen ausgekleidet sind.

Diese Epithelzellen sind insbesondere in den Arterien ständig den tangentialen Scherkräften ausgesetzt, die für die Aufrechterhaltung der Endothelfunktion einschließlich des Gefäßtonus durch die Synthese von Stickstoffmonoxid (NOₓ), Blutgerinnung, Entzündungsreaktion, Arteriosklerose, Angiogenese und Apoptose unerlässlich sind.

Extrakorporale Zirkulation (abgekürzt CEC) ist ein medizinisches Verfahren, das zum Ersatz von Herz und Lunge in Operationssälen, Herzkatheterlabors oder Intensivstationen bei Patienten eingesetzt wird.

CEC ist notwendig, um die Organperfusion und Stoffwechselfunktionen während chirurgischer Eingriffe aufrechtzuerhalten oder um den versagenden Herzmuskel bis zur Genesung oder als Relais vor der Transplantation zu unterstützen.

Je physiologischer der Blutfluss, desto weniger sind Begleitschäden in den Organen zu erwarten. Wenn ein Mensch auf den Blutfluss einer externen Maschine angewiesen ist, z.B. bei Herz- oder Gefäßoperationen, kommt es bei den aktuell auf dem Markt befindlichen Geräten zu einer unphysiologischen Perfusion der Organe. Trotz der Fortschritte, die auf dem Gebiet der CEC seit ihrem Aufkommen in den frühen 1950er Jahren erzielt wurden, weist sie immer noch Nachteile auf.

Der laminare Fluss der konventionellen CEC befreit das Endothel von tangentialen Kraftreizen der Scherbeanspruchung, was zu einem endothelialen Dysfunktionssyndrom führt, das für die Aktivierung der Komplementkaskade, Entzündungsreaktion, Apoptose und hämodynamische Störungen verantwortlich ist, insbesondere bei Neugeborenen und Kindern. Diese unphysiologische Perfusion führt zwar zunächst zu nur geringen Schäden in den versorgten Organen. Je länger jedoch die Perfusion anhält, desto ausgeprägter wird der Schaden. Mit der Konversion des Blutflusses einer Herz-Lungen-Maschine in einen physiologischeren Blutfluss, könnte die blutflussassoziierte Organschädigung abgeschwächt oder sogar verhindert werden.

### Stand der Technik

Mit den bekannten intraoperativ eingesetzten Herzlungenmaschinen (HLM) kann zurzeit kein physiologischer Blutfluss aufgebaut werden. Die Rotationspumpen bzw. Zentrifugalpumpen erzeugen in der Regel einen gleichmäßigen laminaren Blutstrom ohne Pulswellen. Die Verdrängerpumpen erzeugen hingegen schon einen pulsatilen Blutstrom mit kleiner und schneller Oszillation. Diese Art des Blutflusses erinnert eher an den Blutstrom, der bei einem schnellen Herzkammerflattern erzeugt wird. Eines der sensibelsten Organe diesbezüglich ist der Darm. Durch die Schädigung des Darmes entsteht eine systemisch inflammatorische Reaktion (SIRS), die auch andere Organsysteme in Mitleidenschaft zieht und den Patienten schwer erkranken lassen kann. Eine verlängerte postoperative intensivmedizinische Überwachung und Behandlung können erforderlich werden. Somit verlängert sich der gesamte stationäre Aufenthalt und die komplette Rehabilitation des Patienten deutlich.

Die HLM induzierte Darmschädigung hängt nach aktueller Datenlage nicht an der Blutmenge, die durch den Darm fließt, sondern an der Fehlverteilung des Blutes in den Darmschichten. Mögliche Ursache hierfür könnte der fehlende pulsatile Reiz des Blutstromes auf die Schließmuskeln der Arteriolen (kleinste Arterien) sein. Deshalb ist es ein Nachteil des bestehenden Standes der Technik, dass die extrakorporale Zirkulation keinen pulsativem Blutfluss, wie er natürlicherweise auftritt, aufweist.

### Aufgabe

Aufgabe der vorliegenden Erfindung ist es eine Vorrichtung bereitzustellen, welche einen pulsatilen Fluss in die arterielle Strombahn abgeben kann, der im Idealfall einer physiologischen Perfusion aus dem Herzen entspricht.

### Lösung der Aufgabe

Diese Aufgabe wird erfindungsgemäß gelöst durch eine Vorrichtung für den Einsatz in einer Herzlungenmaschine, die eine extrakorporale Zirkulation mit pulsativem Blutfluss ermöglicht.

Die Vorrichtung 1 zur Erzeugung einer extrakorporalen Zirkulation mit pulsativem Blutfluss umfasst dabei wenigstens:
**Rohr** 10:Das Rohr 10 ist so ausgebildet, dass es an eine Herzlungenmaschinen anschließbar ist und Blut aufnehmen kann, sodass ein externer Blutdurchfluss durch das Rohr 10 und so eine extrakorporale Zirkulation erfolgen kann. Das Rohr ist dabei vorzugsweise aus Polyurethan, PVC oder Polyethylen.

Dazu weist das Rohr 10 dabei an einem Ende eine erste Öffnung 110 und an seinem gegengesetzten Ende eine zweite Öffnung 120 auf. Zur Verbindung des Rohrs 10 mit einer Herzlungenmaschine weist die Vorrichtung vorzugsweise an jeder der Öffnungen 110,120 eine Kanüle 50,55 auf. Die Öffnungen 110,120 sind vorzugsweise so ausgebildet, dass das Verbinden z.B. über Steckverbindungen erfolgen kann. Das Verbinden erfolgt dabei indem, wie bei Kanülen üblich, indem ein von der Herzlungenmasche ausgehender Schlauch mit dem Rohr 10 zusammengesteckt wird. Durch entsprechende Reibung wird die Verbindung gehalten. Übliche Verbindungen erfolgen beispielsweise über 3/8 Konnektoren aus Polycarbonat.

Das wenigstens eine Rohr 10 umfasst dabei ein Reservoir 130. Dieses ist zumindest abschnittsweise elastisch ausgebildet, sodass es Blut kurzzeitig aufstauen und zur Verfügung stellen kann, wenn Blut mit einer fluktuierenden Rate durch das Rohr 10 transportiert wird. Das Reservoir 130 ist dabei vorzugsweise als ein elastischer Kunststoffschlauch ausgebildet. Das verwendete Material des Reservoirs muss dabei ellastischer ist als das Material des Rohrs sein, sodass die Ausdehnung des Reservoirs mit steigendem Druck zunimmt, ohne dass sich das Rohr 10 ausdehnt. Ein geeignetes Material ist dabei Polyurethan. Dabei ist es auch möglich das gleiche Material wie im Rohr zu verwenden. Das Reservoir soll verhindern, dass Luft (insbesondere aus dem Oxygenator einer HLM) in die Blutbahn gesaugt wird, weil bei einer höheren Flussrate mehr Blut durch die Vorrichtung 1 transportiert wird.

### Ventil 20

Das Ventil 20 ist eine Armatur zur stetigen Dosierung (Steuerung) bis hin zur Absperrung des Durchflusses von Blut durch das Rohr 10.

Das Ventil 20 ist dabei zwischen dem Reservoir 130 und der zweiten Öffnung 120 des Rohrs 10 angeordnet. Dabei ist es so im Rohr 10 ausgebildet, dass es die Menge des Blutes, welches durch das Rohr 10 strömt, steuern kann indem es den durchströmbaren Rohrdurchmesser verändern kann. Das Ventil 20 ist dabei vorzugsweise als Ringventil ausgeführt. Ein gängiges Material für Ringventile ist Polycarbonat. Vorzugsweise ist das Ventil dabei so ausgebildet, dass es kegelförmig mit Kegelspitze in Richtung der Öffnung 120 ausgerichtet ist. Dabei ist die Eintrittsfläche des Kegels vorzugsweise doppelt so groß wie seine Eintrittsfläche ausgebildet. Dies führt zu einem besonders gleichmäßigen Blutfluss. Weiterhin umfasst das Ventil 20 eine Datenschnittstelle 250 um Steuerdaten aus einem Steuerungselement 40 empfangen zu können, damit es gesteuert werden kann.

### Pumpe 30

Die Pumpe 30 ist dabei vorzugsweise als Zentrifugalpumpe ausgebildet. Sie ist dabei zwischen dem Ventil 20 und der zweiten Öffnung 120 des Rohrs 10 angeordnet. Sie kann dabei im Rohr 10 oder außen am Rohr 10 angeordnet. Die Pumpe 30 ist so ausgebildet, dass sie die Flussrate und damit den Blutdruck des Blutes, welches das Rohr 10 passiert kann, verändern kann. Weiterhin umfasst die Pumpe 30 eine Datenschnittstelle 350 um Steuerdaten aus einem Steuerungselement 40 empfangen zu können, damit sie gesteuert werden kann.

### Steuerelement 40

Das Steuerelement 40, ist dabei so ausgebildet, dass es die Pumpe 30 und das Ventil 20 im Rohr 10 steuern kann. Dazu ist das Steuerelement 40 so ausgebildet, dass es verschiedene Messdaten, welche es von externen Sensoren beispielsweise innerhalb einer Herzlungenmaschine nutzen kann. Diese Messdaten von externen Sensoren können dabei beispielsweise sein:
a) EKG-Rhythmus des Patienten
b) Flussmenge an Blut, die der extrakorporale Kreislauf in den Oxygenator einer HLM transportiert
c) Flussmenge die hinter dem Oxygenator einer HLM ankommt

Alternativ oder zusätzlich kann das Steuerelement 40 Messdaten von Sensoren innerhalb der Vorrichtung 1 selbst nutzen. Diese können sein:
d) Blutdruck im Rohr 10 zwischen dem Ventil 20 und der Pumpe 30
e) zeitliche Verlauf des Blutdrucks im Rohr an dessen zweiter Öffnung 120
f) Füllstand des Reservoirs 130

Das Steuerelement ist dabei so ausgebildet, dass es vorzugsweise folgende Parameter steuern kann.
i. Frequenz und Amplitude der Pumpe 30 um den Verlauf und die Höhe des durch die Vorrichtung 1 erzeugten Blutdrucks zu regeln.
ii. Länge der Beschleunigungsphase der Pumpe 30 gegebenenfalls in Abhängigkeit von EKG- Messdaten
iii. Öffnungs- und Schließzeiten des Ventils 20 nach Volumenbedarf um den Füllstand des Reservoirs 130 zu regeln.

### Eine Datenschnittstelle 450:

Die Datenschnittstelle 450 des Steuerelements 40 ist so ausgebildet, dass sie die Messdaten aus einem externen Datenverarbeitungssystem (z.B. einer Herzlungenmaschine) oder von Sensoren innerhalb der Vorrichtung 1 empfangen und diese Daten an eine Auswerteeinheit 420 weiterleiten kann. Weiterhin ist die Datenschnittstelle 450 des Steuerelements 40 so ausgebildet, dass sie Zieldaten (Sollwerte) für verschiedene Messdaten empfangen kann.

Die Datenübermittlung kann dabei über eine feste Datenleitung (z.B. über ein LAN etc.), oder drahtlos (z.B. über WLAN, Bluetooth etc.) erfolgen.

Weiterhin ist die Datenschnittstelle 450 so ausgebildet, dass sie verschiedene Daten an die Auswerteinheit 420 übermitteln kann. Die Datenübermittlung kann dabei über eine feste Datenleitung (z.B. über ein LAN etc.), oder drahtlos (z.B. über WLAN, Bluetooth etc.) erfolgen.

Die Datenschnittstelle 450 ist üblicherweise auf den Austausch mit verschiedenen externen Datenverarbeitungssystemen ausgelegt, d.h. gängige Schnittstellen zum Datenaustausch sind bspw. USB, Cardreader, LAN, WLAN, Bluetooth.

Die Datenschnittstelle 450 ist dabei so ausgebildet, dass ein Anwender mit einem beliebigen webfähigen Gerät (bspw. Tablet, PC, Smartphone) im lokalen Netzwerk über die Datenschnittstelle 450 auf das Steuerelement 40 der Vorrichtung 1 zugreifen kann.

### Eine Auswertungseinheit 420:

Die Auswertungseinheit 420 ist so ausgebildet, dass sie die Messdaten von der Datenschnittstelle 450 empfangen kann. Die Datenübermittlung kann dabei über eine feste Datenleitung (z.B. über eine interne Datenleitung, LAN etc.), oder drahtlos (z.B. über WLAN, Bluetooth etc.). Als Auswertungseinheit 20 dient dabei ein Gerät zur elektronischen Datenverarbeitung, z.B. ein in die Vorrichtung 1 integrierter programmierbarer Mikroprozessor.

Die Auswertungseinheit ist so ausgebildet, dass sie aus dem Vergleich von Messdaten und Zieldaten Steuerdaten erzeugen kann.

Die Auswertungseinheit 420 ist weiterhin so ausgebildet, dass sie die Steuerdatendaten an die Pumpe 20 und das Ventil 30 übermitteln kann. Die Datenübermittlung kann dabei über eine feste Datenleitung (z.B. über eine interne Datenleitung, LAN etc.), oder drahtlos (z.B. über WLAN, Bluetooth etc.) erfolgen.

### Weitere Ausführungsformen:

In einer Weiterführung der erfindungsgemäßen Vorrichtung weist das Rohr 10 an seiner zweiten Öffnung 120 einen Drucksensor auf.

In einer Weiterführung der erfindungsgemäßen Vorrichtung 1 ist das Rohr 10 gerade ausgeführt. Alternativ ist das Rohr 10 abgewinkelt ausgebildet. Dabei ändert es seine Ausrichtung an einem Abknickpunkt A um einen Winkel α. α beträgt vorzugsweise zwischen 75° und 105°.

In einer weiteren Ausführungsform weist die Vorrichtung an der zweiten Öffnung 120 des Rohrs 10, eine gewinkelte Kanüle 55 auf, die eine Perfusion in 2 Richtungen ermöglicht. Dabei ist Kanüle 55 vorzugsweise als separates Bauelement ausgeführt, welches mit dem Rohr 10 verbindbar ist. Hierbei weist die gewinkelte Kanüle 55 vorzugsweise einen Sensor auf, um den Blutdruck zu messen. Alternativ weist die gewinkelte Kanüle 55 ein paralleles weiteres Rohr auf, das so ausgebildet ist, dass durch dieses Röhrchen Blut zu einem externen Sensor, zur Messung des Blutdrucks fließen kann und über einen separaten Drucksensor die Daten liefert. Die Kanüle 55 ist vorzugsweise dabei zu ihrer Spitze hinzulaufend. Weiterhin ist sie an einem Abknickpunkt B an der Spitze um einen Winkel β abgewinkelt. β beträgt vorzugsweise zwischen 75° und 105°. Dabei weist diese Kanüle 55, eine Innenkanüle 550 auf. die in ihrem Inneren angeordnet ist. und einen und von β abweichenden Winkel γ gewinkelt ist, sodass Spitze der Innenkanüle 550 durch eine weitere Öffnung 555 aus der Kanüle 55 heraustreten kann. Ein Winkel γ=0° bedeutet, dass die Innenkanüle 550 parallel zum Rohr 10 ist. Die Kanüle 55 weist dabei eine Seitenöffnung auf, welche die Perfusion durch die äußere Winkelkanüle 55ermöglicht. Die Innenkanüle 550 ist auf der der Öffnung 120 zugewandten Seite der Kanüle zugewandten Seite des Abknickpunkts fixiert. Die Spitze der Innenkanüle 550 ist dabei so ausgebildet, dass sie durch eine Öffnung 555 der Kanüle 55 heraustreten kann. Die Öffnung der inneren Kanüle 550 ist so groß, dass die Position der Winkelkanüle 55 auf die optimale Rotation ausgerichtet werden kann. Damit bilden die Kanüle 55 und die Innenkanüle 550 eine Zweiwegekanüle. Diese Zweiwegekanüle hat die Möglichkeit, dass ein zweites gewinkeltes Ende d.h.die Spitze 559 der Innenkanüle 550 heraustreten, welches Blut in die entgegengesetzte Flussrichtung transportieren kann. In dieser Ausführungsform kann die Vorrichtung in zwei unterschiedlichen Richtungen Blut transportieren. Dies ist wichtig um bei der extrakorporalen Zirkulation Blut entnehmen oder hinzugeben zu können.

### Abbildungslegenden und Bezugszeichenliste

Die Pfeile 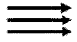 zeigen in allen Abbildungen die bevorzugte Richtung, in welcher die extrakorporale Zirkulation erfolgen soll.

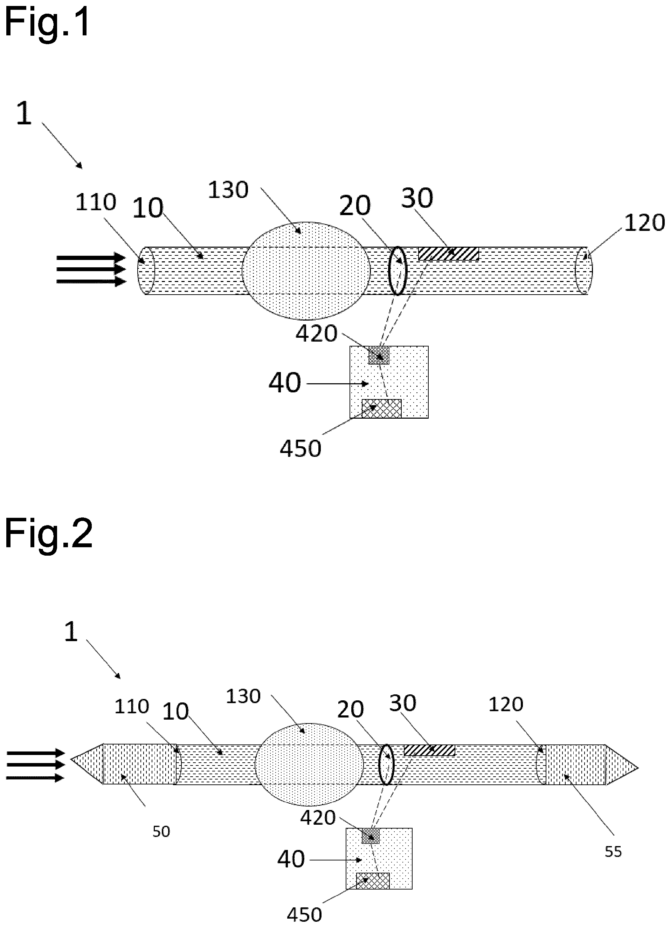

Fig. 1 zeigt schematisch eine gerade Ausführungsform der erfindungsgemäßen Vorrichtung 1
Fig.2 zeigt schematisch eine gerade Ausführungsform der erfindungsgemäßen Vorrichtung 1. Das Rohr 10 weist an der ersten Öffnung 110 eine Kanüle 50 und an der zweiten Öffnung 120 eine weitere Kanüle 55 zur Verbindung mit einer Herzlungenmaschine auf.

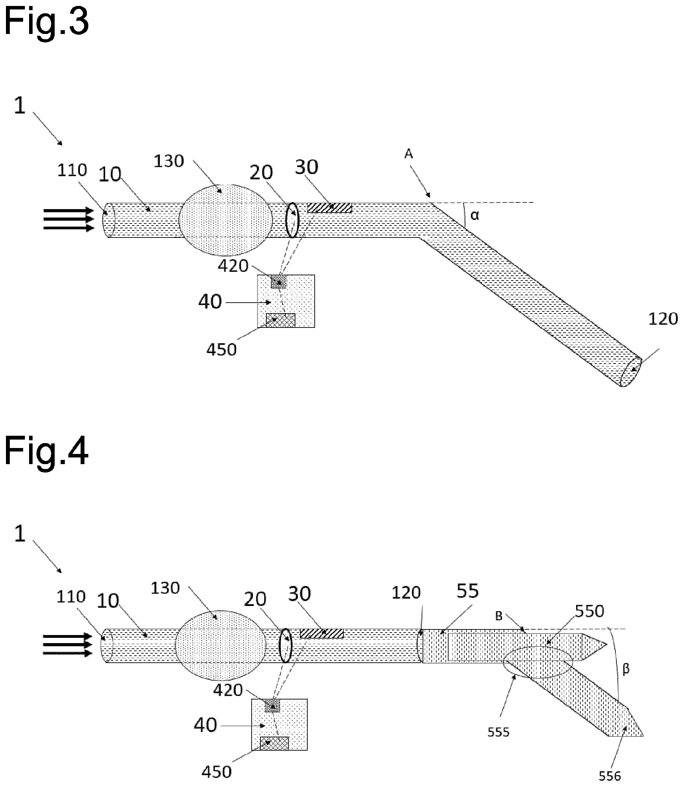

Fig. 3 zeigt schematisch eine um einen Winkel α abknickende Ausführungsform der erfindungsgemäßen Vorrichtung 1
Fig.4 zeigt schematisch eine Ausführungsform der erfindungsgemäßen Vorrichtung 1 mit einer Kanüle 55, welche an einer Abknickstelle B um einen Winkel ihre Richtung ändert und eine Innenkanüle 550 aufweist. In dieser Abbildung ist diese Innenkanüle 550 herausgefahren.

### Bezugszeichen

1 Vorrichtung
10 Rohr
110 erste Öffnung
120 zweite Öffnung
130 Reservoir
20 Auswertungseinheit
30 Pumpe
40 Steuerelement
50,55 Kanüle
550 Innenkanüle
555 Öffnung
556 Spitze der Innenkanüle
420 Auswerteinheit
450 Datenschnittstelle
A Abknickpunkt Rohr
B Abknickpunkt Kanüle
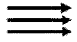 bevorzugte Flussrichtung

## Patentansprüche

1. Vorrichtung (1) zur Erzeugung einer extrakorporalen Zirkulation mit pulsativem Blutfluss wenigstens umfassend
• ein Rohr (10), wobei das Rohr (10) dabei an einem Ende eine erste Öffnung (110) und am entgegensetzten Ende eine zweite Öffnung (120) aufweist, sodass die Vorrichtung (1) an eine Herzlungenmaschinen anschließbar ist und Blut aufnehmen kann, sodass ein externer Blutdurchfluss durch das Rohr (10) und so eine extrakorporale Zirkulation erfolgen kann,
• ein Ventil (20), welches zwischen der ersten Öffnung (110) und der zweiten Öffnung (120) des Rohrs (10) angeordnet ist,
• eine Pumpe (30), welche zwischen dem Ventil (20) und der zweiten Öffnung (120) des Rohrs (10) angeordnet ist, wobei die Pumpe (30) im Rohr (10) oder außen am Rohr (10) angeordnet ist
• ein Steuerelement (40), welches so ausgebildet ist, dass es die Pumpe (20) und das Ventil (30) im Rohr (10) steuern kann
**dadurch gekennzeichnet, dass**
das Rohr (10) ein Reservoir (130) umfasst, welches zumindest abschnittsweise elastisch ausgebildet ist, sodass es Blut aufstauen und zur Verfügung stellen kann, wenn Blut mit einer fluktuierenden Rate durch das Rohr (10) transportiert wird, wobei das Ventil (20) so ausgebildet ist, dass es die Menge des Blutes, welches durch das Rohr (10) strömen kann, steuert indem es den durchströmbaren Rohrdurchmesser verändert und die Pumpe (30) ausgebildet ist, dass sie die Flussrate und damit den Blutdruck des Blutes, welches das Rohr (10) passieren, verändert, sodass das Ventil (20) und die Pumpe (30) zusammen die Menge des Blutes, welches durch die Rohr (10) fließen kann, regelt, sodass ein pulsativem Blutfluss erzeugt wird.

2. Vorrichtung (1) zur Erzeugung einer extrakorporalen Zirkulation gemäß Anspruch 1 **dadurch gekennzeichnet, dass** das Ventil (20), zwischen dem Reservoir (130) und der zweiten Öffnung (120) des Rohrs (10) angeordnet ist,

3. Vorrichtung (1) zur Erzeugung einer extrakorporalen Zirkulation gemäß Anspruch 1 oder 2 **dadurch gekennzeichnet, dass** das Ventil (20) so ausgebildet, dass es kegelförmig mit Kegelspitze in Richtung der Öffnung (120) ausgerichtet ist.

4. Vorrichtung (1) zur Erzeugung einer extrakorporalen Zirkulation gemäß Anspruch 1,2 oder 3 **dadurch gekennzeichnet, dass** das Rohr (10) an seinem zweiten Ende (120) einen Drucksensor aufweist.

5. Vorrichtung (1) zur Erzeugung einer extrakorporalen Zirkulation gemäß einem der vorigen Ansprüche **dadurch gekennzeichnet, dass** das Rohr (10) gerade ausgeführt ist.

6. Vorrichtung (1) zur Erzeugung einer extrakorporalen Zirkulation gemäß einem der Ansprüche 1 bis 4 **dadurch gekennzeichnet, dass** das Rohr (10) abgewinkelt ausgeführt ist und an einem Abknickpunkt (A) seine Richtung um einen Winkel α ändert.

7. Vorrichtung (1) zur Erzeugung einer extrakorporalen Zirkulation gemäß Anspruch 6 **dadurch gekennzeichnet, dass** α zwischen 75° und 115° beträgt.

8. Vorrichtung (1) zur Erzeugung einer extrakorporalen Zirkulation gemäß einem der vorigen Ansprüche **dadurch gekennzeichnet, dass** sie an der ersten Öffnung (110) eine Kanüle (50) und/ oder an der zweiten Öffnung (120) eine Kanüle (55) zur Verbindung mit einer Herzlungenmaschine aufweist.

9. Vorrichtung (1) zur Erzeugung einer extrakorporalen Zirkulation gemäß Anspruch 8 **dadurch gekennzeichnet, dass** die Vorrichtung an der zweiten Öffnung (120) des Rohrs 10, eine an einem Abknickpunkt B an der Spitze um einen Winkel β gewinkelte Kanüle (55) aufweist,

10. Vorrichtung (1) zur Erzeugung einer extrakorporalen Zirkulation gemäß Anspruch 9 **dadurch gekennzeichnet, dass** die Kanüle (55), eine Innenkanüle (550) aufweist,
wobei die Innenkanüle (550) im Inneren der Kanüle (55) angeordnet ist und auf der der Öffnung (120) zugewandten Seite der Kanüle (55) zugewandten Seite des Abknickpunkts B fixiert ist,
wobei die Innenkanüle (550) um von β abweichenden Winkel γ gewinkelt ist, sodass sie durch eine Öffnung (555) der Kanüle (55) heraustreten kann, wobei die Kanüle (55) und die Innenkanüle (550) eine Zweiwegekanüle bilden durch welche Blut in zwei unterschiedlichen Richtungen transportiert kann, um bei der extrakorporalen Zirkulation Blut dem Blutkreislauf entnehmen oder Flüssigkeit hinzugeben zu können.
